# EUROPÄISCHE PATENTSCHRIFT

(11) **EP 0 411 276 B1**
(45) Veröffentlichungstag und Bekanntmachung des Hinweises auf die Patenterteilung: **02.02.1994**
(21) Anmeldenummer: 90110662.5
(22) Anmeldetag: 06.06.1990
(51) Int. Cl.: C07D 239/66

(54) **Verfahren zur Herstellung von 2-(Methylthio)barbitursäure**
Process for preparation of 2-(Methylthio)-barbituric acid
Procédé de préparation de l'acide 2-(Méthylthio)barbiturique

(30) Priorität: 03.08.1989 DE 3925687
(43) Veröffentlichungstag der Anmeldung: 06.02.1991
(73) Patentinhaber: HÜLS AKTIENGESELLSCHAFT, 45764 Marl (DE)
(72) Erfinder: Feld, Marcel, Dr., D-5000 Köln 90 (DE); Goerentz, Friedrich, D-5216 Niederkassel 2 (DE)

(56) Entgegenhaltungen:
- FR-A- 2 222 376
- US-A- 4 199 583
- THE JOURNAL OF ORGANIC CHEMISTRY, vol. 26, no. 3, März 1961, Seiten 792-803, Columbus, Ohio, US; H.C. KOPPEL et al.: "Pyrimidines. I. Synthesis of pyrimidinethiols"
- REAGENTS FOR ORGANIC SYNTHESIS, L.F. FIESER et al., vol. 1, 1967, John Wiley and Sons Inc., New York, US

## Beschreibung

Die vorliegende Erfindung beschreibt ein Verfahren zur Herstellung von 2-(Methylthio)barbitursäure, die als Zwischenprodukt z.B. für die Gewinnung von Pflanzenschutzmitteln von Interesse ist.

Die erfindungsgemäße Herstellung der 2-(Methylthio)barbitursäure erfolgt durch Umsetzung der wäßrigen Lösung oder Suspension des Na-Salzes der 2-Thiobarbitursäure mit Methylbromid bei milden Temperaturen von unter 90 °C und erhöhtem Druck von 1,5 bis 5 bar.

Aus der Literatur ist die Herstellung von 2-(Methylthio)barbitursäure durch Methylierung des Na-Salzes der 2-Thiobarbitursäure bekannt. Als Methylierungsreagenzien werden dabei einerseits Dimethylsulfat (DE-OS 24 12 854, Beispiel 12; DE-OS 25 23 324, Beispiel c), andererseits Methyljodid (US-OS 4 199 583) verwendet. Trotz Verwendung dieser besonders reaktiven Alkylierungsmittel waren die bei dem beschriebenen Verfahren erzielten Ausbeuten von 33 % (nach DE-OS 25 23 324) bzw.89,3 %(nach US-PS 4 199 583) bei unzureichender Reinheit noch unbefriedigend.
Dimethylsulfat und Methyljodid erfordern wegen der Giftigkeit aufwendige Sicherheitsmaßnahmen und sollten vermieden werden.

Es bestand somit die Aufgabe, ein Verfahren zur Herstellung von 2-(Methylthio) barbitursäure zu entwickeln, das ohne den Einsatz von Dimethylsulfat oder Methyljodid das Zielprodukt in befriedigender Ausbeute und hoher Reinheit liefert.

Diese Aufgabe wird durch das erfindungsgemäße Verfahren, bei dem die Methylierung mit Methylbromid erfolgt, erfüllt.

Es wurde gefunden, daß eine wäßrige Lösung des Na-Salzes der Thiobarbitursäure bereits bei milden Temperaturen mit Methylbromid umgesetzt werden kann. Dies war angesichts der in den beschriebenen Verfahren mit den besonders wirksamen Methylierungsmitteln Dimethylsulfat und Methyljodid erzielten Ergebnissen überraschend. Als bedeutsam hat sich beim erfindungsgemäßen Verfahren für das Erzielen einer hohen Ausbeute und hohen Reinheit das Einhalten eines relativ engen Temperaturbereiches bei erhöhtem Druck erwiesen.

Es konnte gezeigt werden, daß 2-(Methylthio)barbitursäure in Gegenwart von Wasser bei Temperaturen von über 80 °C in erheblichem Maße unter Bildung von Methylmercaptan zersetzt wird, wodurch neben Ausbeuteverlusten und Produktverunreinigungen auch Probleme der Geruchsbelästigung resultieren.

Andererseits ist erwartungsgemäß die Reaktionsgeschwindigkeit der Umsetzung einer wäßrigen Lösung von Na-Thiobarbiturat mit Methylbromid unter Normaldruck bei milden Temperaturen für eine technische Herstellung der 2-(Methylthio)barbitursäure zu gering.

Die vorliegende Erfindung basiert auf der Beobachtung, daß die Reaktionsgeschwindigkeit der Umsetzung einer wäßrigen Lösung bzw. Suspension eines Alkali - und/oder Erdalkalisalzes der 2-Thiobarbitursäure mit Methylbromid bei milden Temperaturen von z.B. Raumtemperatur bereits durch einen geringfügig erhöhten Druck, wie er beispielsweise dem Dampfdruck des Methylbromids bei diesen milden Temperaturen entspricht, in überraschend starkem Maße gesteigert wird.

Das erfindungsgemäße Verfahren beschreibt somit die Herstellung von 2-(Methylthio)barbitursäure, dadurch gekennzeichnet, daß die wäßrige Lösung und/oder Suspension eines Alkali- und/oder Erdalkalisalzes der 2-Thiobarbitursäure bei Temperaturen von 20 bis 80 °C, vorzugsweise bei 20 bis 60 °C, und einem erhöhten Druck von 1,5 bis 5 bar umgesetzt wird. Vorzugsweise ist der Druck der Dampfdruck von Methylbromid.

Nach dem erfindungsgemäßen Verfahren kann 2-(Methylthio)barbitursäure in Reaktionszeiten von weniger als 10 h mit hoher Reinheit von über 99 % in Ausbeuten von mehr als 90 % d.Th. unter technisch einfachen Bedingungen gewonnen werden. Nach beendeter Umsetzung braucht die in Wasser nahezu unlösliche 2-(Methylthio)barbitursäure lediglich durch eine übliche Fest-Flüssig-Trennung abgetrennt zu werden. Um Zersetzungen des Zielproduktes während der Trocknung bei erhöhten Temperaturen zu vermeiden, empfiehlt es sich, das im filterfeuchten Produkt enthaltene Wasser vor der Trocknung durch ein geeignetes Lösemittel oder Lösemittelgemisch zumindest größtenteils zu verdrängen. Als dafür geeignet hat sich beispielsweise Aceton erwiesen.

Die beim erfindungsgemäßen Verfahren eingesetzte wäßrige Lösung bzw. Suspension von Thiobarbitursäuresalzen kann durchaus noch andere, mit Wasser mischbare oder entsprechend der Löslichkeit mit Wasser teilweise mischbare Lösemittel enthalten.So kann beispielsweise ein gemäß bekannten Literaturverfahren durch Umsetzung von Thioharnstoff, Malonsäurediethylester und Natriumethanolat hergestelltes und aus ethanolischer Suspension abgetrenntes Na-Thobarbiturat ohne vorherige Trocknung mit dem im filterfeuchten Produkt enthaltenen Ethanol für die erfindungsgemäße Methylierung zu 2-(Methylthio) barbitursäure in einem dann zwangsläufig ethanolhaltigen wäßrigen Medium eingesetzt werden.

Die erfindungsgemäße Methylierung erfolgt unter Rühren bzw. Umpumpen der Lösung bzw. Suspension. Das Methylbromid kann flüssig oder gasförmig zugeführt werden. Im Falle einer gasförmigen Zuführung des Methylbromids kann dies in die Lösung/Suspension hinein oder in die Gasphase erfolgen, worauf der Druck auf die erforderlichen Werte erhöht wird. Die Umsetzungsgeschwindigkeit von beispielsweise Na-Thiobarbiturat mit Methylbromid und die Löslichkeit des Salzes, aber auch des Zielproduktes, im wäßrigen Medium können durch zusätzliches Alkali erhöht werden. Die 2-(Methylthio)barbitursäure wird in diesem Fall durch Zusatz einer dem Alkaliüberschuß entsprechenden Menge einer geeigneten Säure nach beendeter Umsetzung gefällt. Nachteilig wirkt sich der Alkaliüberschuß allerdings auf die Reinheit der 2-(Methylthio)barbitursäure insofern aus, als unter diesen Bedingungen in kleinen Mengen auch dimethylierte Thiobarbitursäuren gebildet werden. Sofern diese geringen Verunreinigungen bei Nachfolgereaktionen nicht stören, kann die erfindungsgemäße Methylierung auch in Gegenwart eines gegenüber Thiobarbitursäure stöchiometrischen Basenüberschusses von beispielsweise bis zu ca. 3 Äquivalenten durchgeführt werden. Ist dagegen eine 2-(Methylthio)barbitursäure hoher Reinheit erwünscht, so ist ein Basenüberschuß zu vermeiden, indem entweder direkt ein Salz der Thiobarbitursäure oder aber Thiobarbitursäure mit einer äquivalenten Menge einer Base zum Einsatz gebracht werden.

### Beispiel 1

Ein mit 20 g Na-Thiobarbiturat und 100 g H₂0 beschicktes Reaktionsgefäß wurde mit einer CH₃Br-Bombe verbunden, so daß sich im Reaktionsgefäß ein Druck von 1,9 bar einstellte. Unter Rühren wurde auf 45 °C erwärmt und unter Aufrechterhaltung von Druck und Temperatur die Reaktion über einen Zeitraum von 8 h geführt, wobei mit fortschreitender Reaktion mehr und mehr 2-(Methylthio)barbitursäure in Form eines weißen Kristallisates anfiel. Nach beendeter Reaktion wurde der gebildete Feststoff abfiltriert, mit Wasser und Aceton gewaschen und im Vakuum bei allmählich bis auf 100 °C gesteigerter Temperatur getrocknet. Es wurden 17,4 g (91,3 % d.Th.) 2-(Methylthio)barbitursäure mit einer Reinheit von 99,8 % gewonnen.

### Beispiel 2

Der in Beispiel 1 beschriebene Versuch wurde unter Einsatz von 23,4 g eines durch Umsetzung von Thioharnstoff mit Malonsäurediethylester und Natriumethanolat in Ethanol hergestellten, noch 2,3 g Ethanol enthaltenden Na-Thiobarbiturats wiederholt. Dabei wurden nach einer Reaktionszeit von 4 h 18,6 g (92,7 % d.Th.) Zielprodukt gleicher Reinheit wie in Beispiel 1 erhalten.

### Beispiel 3

Der in Beispiel 1 beschriebene Versuch wurde bei 70 °C und 4 h Reaktionszeit wiederholt. Dabei resultierten 16 g (84 % d.Th.) Zielprodukt einer Reinheit von 99,2 %. Die Aufarbeitung war mit erheblicher Geruchsbelästigung verbunden.

### Vergleichsbeispiel A und B

Der in Beispiel 1 beschriebene Versuch wurde bei einer Reaktionstemperatur von 90 °C und Reaktionszeiten von 2 bzw. 6 h wiederholt. Das Zielprodukt resultierte hierbei in Rohausbeuten von 3,6 bzw. 14,7 g mit Reinheitsgraden von nur 33,4 bzw. 44 %.

### Beispiele 4 und 5

Beispiel 1 wurde bei 20 bis 25 °C wiederholt, wobei nach einer Reaktionszeit von 8 h 11,9 g (62,5 % d.Th.) und nach 16 h 17,7 g (93 % d.Th.) Zielprodukt mit einer Reinheit von jeweils 99,8 % erhalten wurden.

### Beispiel 6

Beispiel 1 wurde unter Einsatz von 20 g Na-Thiobarbiturat, 90 g Wasser und 15 g Methanol bei 20 - 25 °C und einer Reaktionszeit von 24 h wiederholt, wobei 17 g (89,5 % d.Th.) Zielprodukt guter Reinheit erhalten wurden.

### Beispiel 7

Beispiel 4 wurde unter Einsatz von 17,3 g (0,12 Mol) 2-Thiobarbitursäure, 9,6 g NaOH (0,24 Mol) und 100 g Wasser wiederholt, wobei bereits nach 4 h 15,5 g (82 % d.Th.) Zielprodukt mit einer Reinheit von 95,7 % nach Ansäuern des Reaktionsgemisches mit H₂S0₄ erhalten wurden.

### Vergleichsbeispiel C

In einem mit einer klaren Lösung von 16,6 Na-Thiobarbiturat in 200 g H₂O beschickten Kolben wurde die Luft über der Flüssigkeit durch Evakuieren und Begasen mit CH₃Br ausgetauscht. Dann wurde bei 20 bis 25 °C und Normaldruck kräftig gerührt, wobei durch ein entsprechendes CH₃Br-Angebot dafür gesorgt wurde, daß durch Umsetzung in der Gasphase ggf. resultierende CH₃Br-Verluste unter Normaldruck stets ausgeglichen worden wären. Nach 19 h wurde gegenüber dem evakuierten Kolben nur eine Gewichtszunahme von weniger als 1 g festgestellt. Die schwach getrübte Lösung enthielt kein durch Filtration isolierbares Produkt und wies lediglich einen Br-Gehalt von 629 mg (= 7,9 % d Th.) auf.

### Beispiel 8

Nach Beendigung des im Vergleichsbeispiel C beschriebenen Versuches wurde in der Gasphase über der dort nach 19 h erhaltenen schwach getrübten Lösung der CH₃Br-Druck auf 1,9 bar erhöht und der Versuch unter ansonsten gleichen Bedingungen bei diesem erhöhten Druck für 4 h fortgeführt. Bereits nach 30 Min. setzte eine mit fortschreitender Reaktion weiter zunehmende Fällung ein. Nach 5 h wurde der Versuch abgebrochen. Die Filtration ergab 12,4 g (78 % d.Th.) 2-(Methylthio)barbitursäure und einen Br-Gehalt im Filtrat von 7,06 g (= 88 % d.Th.).

### Beispiel 9

Eine Suspension von 14,2 g 2-Thiobarbitursäure (0,1 Mol) und 2,9 g CaO (0,05 Mol) in 200 g Wasser wurde entsprechend Beispiel 1 für 20 h mit CH₃Br behandelt. Nach dem Entspannen wurde für die Suspension eine Gewichtszunahme von 12,0 g entsprechend einer CH₃Br-Aufnahme von 0,13 Mol festgestellt. Nach Filtration und Trocknung wurden 13,4 g 2-(Methylthio)barbitursäure einer Reinheit von 96,9 % erhalten (83,3 % d.Th. Ausbeute).

## Patentansprüche

1. Verfahren zur Herstellung von 2-(Methylthio)barbitursäure, dadurch gekennzeichnet, daß eine wäßrige, ggf. auch zusätzliche Basen und/oder andere Lösemittel enthaltende Lösung oder Suspension eines Alkali und/oder Erdalkalisalzes der 2-Thiobarbitursäure bei Temperaturen von unter 80 °C und erhöhtem Druck von 1,5 bis 5 bar mit Methylbromid umgesetzt wird.

2. Verfahren nach Anspruch 1, dadurch gekennzeichnet, daß die Reaktion unter dem Dampfdruck des Methylbromids zwischen Raumtemperatur und der jeweiligen Reaktionstemperatur durchgeführt wird.

3. Verfahren nach Anspruch 1 oder 2, dadurch gekennzeichnet, daß die Reaktion bei einer Temperatur von 20 bis 60 °C durchgeführt wird.

4. Verfahren nach einem der Ansprüche 1 bis 3, dadurch gekennzeichnet, daß ein durch Umsetzung von Thioharnstoff mit einem Malonester und einem Natriumalkoholat im alkoholischen Medium hergestelltes Na-Thiobarbiturat ggf. ohne vorherige Trocknung eingesetzt wird.

## Claims

1. Method for the preparation of 2-(methylthio) barbituric acid, characterised in that an aqueous solution or suspension of an alkali and/or alkaline earth salt of 2-thiobarbituric acid, which solution or suspension optionally also contains additional bases and/or other solvents, is reacted with methylbromide at temperatures of below 80°C and at an increased pressure of 1.5 to 5 bar.

2. Method according to claim 1, characterised in that the reaction is carried out under the vapour pressure of the methylbromide between ambient temperature and the respective reaction temperature.

3. Method according to claim 1 or 2, characterised in that the reaction is carried out at a temperature of 20 to 60°C.

4. Method according to one of the claims 1 to 3, characterised in that a Na-thiobarbiturate, which is prepared by reaction of thiourea with a malonic ester and a sodium alcoholate in alcoholic medium, is used, if applicable, without previous drying.

## Revendications

1. Procédé de préparation de l'acide 2-(méthylthio)barbiturique, caractérisé en ce qu'on fait réagir avec le bromure de méthyle, à des températures inférieures à 80°C et sous une pression élevée de 1,5 à 5 bar, une solution ou une suspension aqueuse, contenant éventuellement aussi des bases supplémentaires et/ou d'autres solvants,un sel d'un métal alcalin et/ou alcalinoterreux de l'acide 2-thiobarbiturique.

2. Procédé selon la revendication 1, caractérisé en ce que la réaction est mise en oeuvre sous la pression de vapeur du bromure de méthyle à une température comprise entre la température ambiante et la température de réaction correspondante.

3. Procédé selon la revendication 1 ou 2, caractérisé en ce que la réaction est mise en oeuvre à une température de 20 à 60°C.

4. Procédé selon l'une des revendications 1 à 3, caractérisé en ce qu'on utilise, éventuellement sans séchage préalable, un thiobarbiturate de sodium préparé par la réaction de la thiourée avec un ester de l'acide malonique et un alcoolate de sodium dans un milieu alcoolique.
